# EUROPÄISCHE PATENTSCHRIFT

(11) **EP 3 017 781 B1**
(45) Veröffentlichungstag und Bekanntmachung des Hinweises auf die Patenterteilung: **12.12.2018**
(21) Anmeldenummer: 15192491.7
(22) Anmeldetag: 02.11.2015
(51) Int. Cl.: A61B 18/04, A61B 18/00, A61B 18/12

(54) **PLASMACHIRURGISCHES GERÄT MIT ABSTANDSHALTER**
PLASMA SURGICAL DEVICE WITH SPACER
APPAREIL DE CHIRURGIE AU PLASMA EQUIPE D'ENTRETOISE

(30) Priorität: 07.11.2014 DE 102014116253
(43) Veröffentlichungstag der Anmeldung: 11.05.2016
(73) Patentinhaber: Ovesco Endoscopy AG, 72074 Tübingen (DE)
(72) Erfinder: Ho, Chi-Nghia, 72074 Tübingen (DE); Anhöck, Gunnar, 72074 Tübingen (DE); Conrad, Gabor, 72074 Tübingen (DE); Schurr, Marc O., 72074 Tübingen (DE); Gottwald, Thomas, 72074 Tübingen (DE)
(74) Vertreter: Winter, Brandl, Fürniss, Hübner, Röss, Kaiser, Polte - Partnerschaft mbB

(56) Entgegenhaltungen:
- WO-A1-2006/084316
- DE-A1-102009 002 278
- DE-B4-102008 004 843
- DE-U1- 9 117 111
- GB-A- 2 495 400
- US-A1- 2006 189 976
- US-A1- 2011 301 412
- US-A1- 2012 041 437

## Beschreibung

Die Erfindung betrifft ein plasmachirurgisches Instrument gemäß dem unabhänigen Anspruch 1.

### Hintergrund der Erfindung

Plasmachirurgische Verfahren sowie Plasmaapplikatoren und dergleichen Einrichtungen/Geräte zu deren Anwendung sind grundsätzlich bekannt. Bereits seit mehr als 50 Jahren wird beispielsweise ein unter der Bezeichnung Fulguration oder Spray-Koagulation bekanntes plasmachirurgisches Verfahren, insbesondere zur thermischen Hämostase, bei chirurgischen Operationen angewendet.

Hierbei wird ein Plasma ausschließlich in Luft erzeugt, d. h. es entstehen vorwiegend Sauerstoffplasma und Stickstoffplasma. Beide Plasmen sind bekanntlich chemisch sehr reaktiv und erzeugen an der Gewebeoberfläche Karbonisationseffekte, Pyrolyseeffekte und folglich auch Vaporisation von Gewebe sowie Rauch. Diese unbeabsichtigten Nebeneffekte der Fulguration bzw. Spray-Koagulation stören bzw. behindern die Anwendung dieses plasmachirurgischen Verfahrens, insbesondere bei endoskopischen Operationen. Im Weiteren wurde daher das Gas im Allgemeinen durch ein Edelgas, insbesondere Argon, ersetzt, um diese Nachteile zu umgehen.

### Stand der Technik

Beispielsweise offenbart die DE 10 2008 004 843 B4 ein Plasmabehandlungsgerät, welches für die flexible Endoskopie genutzt wird.

Durch seinen Aufbau kann mit diesem Gerät eine plasmachirurgische Behandlung innerhalb eines menschlichen Körpers durchgeführt werden. Bei dem bekannten chirurgischen Gerät wird innerhalb eines Endoskops eine Sonde bzw. ein Katheter verlegt bzw. in dessen Arbeitskanal eingeführt, der über eine, im Inneren der Sonde/des Katheters verlegte Anschlussleitung die elektrische Energie von einer hochfrequenten Strom/-Spannungsquelle bzw. von einem Generator zu einer Elektrode am distalen Ende der Sonde/des Katheters leitet.

Im Folgenden Text wird nur noch Bezug auf den Katheter genommen. Die schon genannte Sonde ist analog in ihrer Bedeutung.

An der genannten Elektrode bildet sich mittels der eingeleiteten elektrischen Energie ein Plasmalichtbogen zwischen der Spitze der Elektrode und einem sich in unmittelbarer Nähe befindlichen Körpergewebe/Zielgewebe aus. Die Elektrode befindet sich am distalen Ende der Sonde und ragt somit in Richtung distal über das Endoskop bzw. aus dessen Arbeitskanal vor.

Gemäß der DE 10 2008 004 843 B4 ist zwischen dem distalen Ende der im Katheter verlegten Anschlussleitung und dem proximalen Ende der Elektrode zusätzlich ein Widerstandselement, explizit ein kapazitives Widerstandselement, angeordnet und damit der Elektrode unmittelbar vorgeschaltet. Dieses Widerstandselement hat die Aufgabe, die Leistung, die von der Strom-/Spannungsquelle, im Folgenden "Generator" genannt, geliefert wird und durch das Zielgewebe und damit den menschlichen Körper fließt, zu begrenzen. Durch diese genannte Anordnung wird gewährleistet, dass das Widerstandselement möglichst nahe am proximalen Ende der Elektrode platziert ist. Diese Nähe verhindert einen zu großen Einfluss der im System auftretenden Streukapazitäten (infolge von Leitungsabschnitten zwischen dem Widerstandselement und der Elektrode) auf den Spannungsabfall zwischen Elektrodenspitze und Zielgewebe. Der Strom, der durch das Zielgewebe fließt, wird somit hauptsächlich durch diesen zwischen Anschlussleitung und Elektrode liegenden Widerstand in definierter/definierbarer Weise begrenzt.

Ein Problem, welches bei allen Behandlungsgeräten, die einen Plasmalichtbogen zur Behandlung von Gewebe nutzen, auftritt, tritt auch bei dem chirurgischen Instrument der DE 10 2008 004 843 B4 auf:
Der Lichtbogen bildet sich in einer Gasatmosphäre, bevorzugt wie hier in einer Edelgasatmosphäre wie Argon, aus. Die Gasatmosphäre wird dabei durch die am spitzen distalen Ende der Elektrode auftretende, hohe Feldstärke ionisiert. Das Gas wird hierdurch leitend gemacht und bildet nun ein elektrisch leitfähiges Plasma. Über dieses leitfähige Gas bzw. Plasma fließt ein Strom zwischen dem mit einer Neutralelektrode in elektrischer Verbindung stehendem Zielgewebe und der Spitze der Elektrode. Innerhalb des elektrisch leitenden Plasmas bildet sich dadurch ein Lichtbogen aus, mit dem ein Chirurg das jeweilige Zielgewebe behandeln (koagulieren, schneiden) kann.

Um diesen Lichtbogen erstmalig zu zünden, muss der behandelnde Chirurg die Elektrode so nah an das Zielgewebe halten, dass die Feldstärke an der Elektrodenspitze so hoch wird, dass der Lichtbogen zündet. Der Abstand liegt im Bereich von ca. 2 mm zwischen der Spitze der Elektrode, also deren distalem Ende und dem patientenseitigen Zielgewebe. Hat der Lichtbogen im Plasma einmal gezündet, brennt der Lichtbogen weiter, auch wenn sich der Abstand zwischen Zielgewebe und Elektrode (in Grenzen) verändert.

Das vorstehend genannte Problem ist bei der offenen Plasmachirurgie, wenn diese also nicht innerhalb des menschlichen Körpers ausgeführt wird, nicht so bedeutsam. Problematischer sind die Verhältnisse, wenn das Zielgewebe, welches plasmachirurgisch versorgt werden soll, innerhalb des menschlichen Körpers liegt.

Zur Beobachtung des Eingriffsbereichs bzw. zur optischen Kontrolle der Arbeit, wird im Allgemeinen eine Kamera, die ebenfalls im Endoskop bzw. in einem in dem Endoskop angeordneten Funktionskanal sitzt oder auf andere Weise in den Patienten zur Beobachtung des zu versorgenden Zielgewebes eingeführt wird, eingesetzt. Die Handhabung der plasmachirurgischen Geräte findet "remote", also von außerhalb des Körpers gesteuert, statt. Das verhindert eine direkte optische oder haptische Rückmeldung an den Chirurgen, der sich ganz auf das von der Kamera gelieferte Bild des Behandlungs-/Operationsbereichs verlassen muss.

Da das gesamte Gewebe des Körpers des Patienten auf dem Potential der an ihn angeschlossenen Neutralelektrode liegt, die notwendig ist, um bei der hier verwendeten monopolaren Chirurgie einen geschlossenen Stromkreis zu bekommen, kann bei der offenen (monopolaren) Elektrode des plasmachirurgischen Gerätes des Standes der Technik nicht ausgeschlossen werden, dass sich Gewebe, welches nicht behandelt werden soll, näher an der Elektrode befindet als das gewünschte Zielgewebe. In diesem Fall zündet der Lichtbogen gegen eben dieses näherliegende Gewebe, da sich der Strom nach elektrotechnischen Gesetzen immer den Weg des geringsten elektrischen Widerstandes sucht.

Durch diese "Fehlzündungen" bzw. wenn der Plasmalichtbogen beim Zünden zu nahe und zu lang an das Zielgewebe gehalten wird, kann es zu unerwünschten Effekten am Gewebe kommen. Es kann beispielsweise, je nach Behandlungsstrom, sein, dass der Eingriff tiefer liegendes Gewebe unnötig verletzt oder eine zu große Fläche beeinträchtigt wird oder unerwünschte Koagulation, Verbrennung etc. auftreten.

Die offene Elektrode in der DE 10 2008 004 843 B4 erfordert weiterhin, dass der behandelnde Chirurg den Abstand zum Zielgewebe möglichst konstant hält, da sonst, wie auch bei der erstmaligen Zündung, der Lichtbogen auf ein nicht zur Behandlung vorgesehenes Gewebe überspringen kann. Der Strom über den Lichtbogen ist dabei nicht nur abhängig von dem vor der Elektrode liegenden Widerstandselement, sondern auch vom Abstand der Elektrode vom Zielgewebe. Je kleiner der Abstand, desto höher der Behandlungsstrom und damit die Eindringtiefe.

Weiterhin ist das Widerstandselement welches gemäß der DE 10 2008 004 843 B4 eingesetzt wird, recht komplex. Um das Widerstandselement so weit nach vorne zu setzen wie benötigt, muss es sehr klein gebaut sein, um in die Sonde eingebaut und durch den Arbeitskanal eines Endoskops hindurchgeschoben werden zu können. Mit Standardbauteilen aus der Industrie lässt sich dies bei Sondendurchmessern von um die 2,5 mm nicht realisieren. Die DE 10 2008 004 843 B4 schlägt daher eine besondere Konstruktion vor, wobei die Sondenleitung und Teile der Elektrode selber das Widerstandselement ausbilden. Dies ist zwar theoretisch machbar, aber insbesondere in der Praxis lassen sich die benötigten Widerstandswerte auf diese Weise recht schwierig realisieren, da es auch auf konstante Werte des Widerstandselementes ankommt.

Änderungen in der Kapazität machen sich negativ bei der Arbeit mit dem Instrument bemerkbar. Daher ist auch hier der Wunsch entstanden, die Technik so weiterzuentwickeln, dass Standardbauteile verwendet werden können.

GB-A-2 495 400, US-A-2011 301 412, WO-A-2006 084 316, US-A-2006 189 976 und DE-A-10 2009 002 278 offenbaren plasmachirurgische Instrumente.

DE-U-91 17 111 offenbart ein plasmaschirurisches Instrument bei dem ein Ansatzstück in das distale Ende des Arbeitskanals eines Endoskops eingesetzt ist und der Arbeitskanal als Gaszuführleitung dient. Am distalen Ende des Ansatzstücks ist ein Distanzfinger vorgesehen, um einen minimalen Abstand zwischen der Düsenöffnung und dem zu koagulierenden Gewebe zu bestimmen.

### Kurzbeschreibung der Erfindung

Der Erfindung liegt die Aufgabe zugrunde, ein plasmachirurgisches Gerät so weiterzubilden, dass zum Einen verhindert wird, dass der Plasmalichtbogen gegen gesundes, nicht zur Behandlung vorgesehenes Körpergewebe in Form von parasitären Lichtbögen gezündet wird. Gemäß einem weiteren Aspekt der hier vorliegenden Anmeldung soll dem behandelnden Chirurgen weiterhin zu jeder Zeit eine zuverlässige Zündung des Lichtbogens möglich sein. Dies soll auch unter den schwierigen Bedingungen, wenn sich die Behandlungsstelle innerhalb des menschlichen Körpers befindet und der Plasmaapplikator von außen bedient werden muss, gewährleistet sein. Schließlich ist es ein bevorzugtes Ziel der vorliegenden Erfindung, ein plasmachirurgisches Gerät bereit zu stellen, bei welchem im Wesentlichen funktionsoptimierte Bauteile insbesondere für das Widerstandselement und/oder die Elektrode verwendbar sind und das trotzdem problemlos durch den Arbeitskanal eines Endoskops mit standardisiertem Durchmesser (ca. 5mm) hindurchschiebbar ist.

Die Lösung dieser Aufgabe erfolgt durch ein chirurgisches Instrument gemäß dem unabhängigen Patentanspruch 1. Weitere vorteilhafte Ausbildungen sind Gegenstand der Unteransprüche.

Der Grundgedanke der vorliegenden Erfindung besteht in der Bereitstellung eines Abstandshalters (als ein separates Bestandteil der erfindungsgemäßen chirurgischen Geräts/Instruments), der dafür angepasst ist, um als ein Zusatzbauteil an das distale Ende einer Zuführeinrichtung beispielsweise der Endoskopbauart wahlweise montiert zu werden. Der Abstandshalter oder Aufsatz hat zunächst die Funktion, das distale Ende der Zuführeinrichtung in einem bestimmten Abstand zu einem patientenseitigen Zielgewebe zu halten, wenn der Abstandshalter am Zielgewebe an einem dafür vorgesehenen Anlageabschnitt des Abstandshalters aufliegt.

Des Weiteren kann im Abstandshalter die Elektrode bereits montiert sein, welche mit einem Anschluss ausgebildet ist, an den ein durch den Arbeitskanal der Zuführeinrichtung geschobenes, elektrisches Kabel anschließbar ist. In diesem Fall kann die Form der Elektrode entsprechend ihrer Funktion optimiert werden, da sie selbst nicht durch den Arbeitskanal geschoben werden muss. Alternativ hierzu kann der Abstandshalter aber auch mit einer Elektrodenpositioniereinrichtung versehen sein, an der sich eine durch den Arbeitskanal der Zuführeinrichtung geschobene Elektrode (selbsttätig) positioniert und so die richtige Relativlage zu dem Anlageabschnitt des Abstandshalters annimmt.

Schließlich kann der Abstandshalter auch die Funktion der Halterung eines Widerstandselements übernehmen. In diesem Fall kann die Elektrode und der aus dem Stand der Technik per se bekannte Widerstand innerhalb des Abstandshalters montiert sein, wobei in diesem Fall der Wiederstand mit dem Anschluss für das durch den Arbeitskanal der Zuführeinrichtung zu schiebenden Kabel versehen ist.

Konkreter ausgedrückt wird erfindungsgemäß eine Art Aufsatz für eine Zuführeinrichtung (Endoskop) vorgeschlagen, mit welchem ein chirurgisches Instrument zur Behandlung eines Zielgewebes/Körpergewebes mit einem Plasmalichtbogen (monopolarer Lichtbogenaufbau) einführbar ist, wobei der Aufsatz am distalen Ende der Zuführeinrichtung (des Endoskops) angebracht oder anbringbar ist. Der Aufsatz ist an der Zuführeinrichtung (dem Endoskop) bzw. an dessen (Endoskop-) Kopf angebracht oder anbringbar und insbesondere an seinem distalen Abschnitt kappenförmig ausgebildet. Die Oberfläche (Umfangswand) des insgesamt kappenförmigen Aufsatzes bzw. dessen Aufsatzwand umschließt dabei ein Raumvolumen, um mit einem Gas, vorzugsweise ein Edelgas wie Argon, befüllt zu werden.

Der Aufsatz kann vorzugsweise zweigeteilt sein und hierfür einen ersten kappenförmigen Teil am distalen Endabschnitt aufweisen. Vorteilhafter Weise ist das distale Ende des Aufsatzes (das erste kappenförmige Teil) zumindest nach Außen hin verrundet bzw. konvex ausgewölbt. Weiterhin kann der Aufsatz einen zweiten Teil an seinem proximalen Endabschnitt aufweisen. Dieser zweite Teil am proximalen Endabschnitt dient als Montageeinrichtung/Adapter und ist am distalen Ende der Zuführeinrichtung (des Endoskops) befestigt oder ist dafür ausgebildet, um daran befestigt werden zu können.

In einem bevorzugten Ausführungsbeispiel sind beide genannten Teile des Aufsatzes separat zueinander ausgebildet und können miteinander verbunden (gesteckt, verrastet, etc.) werden. Alternativ ist es aber auch möglich, die beiden genannten Teile quasi als Funktionsabschnitte stoffeinstückig auszubilden. Der Aufsatz besteht vorzugsweise aus einem elektrisch nicht leitfähigen, in anderen Worten also aus einem elektrisch isolierenden Material, wie ein Kunststoff oder ein Keramikmaterial.

Innerhalb des Raumvolumens des Aufsatzes ist eine Elektrode angeordnet oder kann darin angeordnet werden. Die Elektrode weist ein distales Ende und ein proximales Ende auf. Gewöhnlich ist das proximale Ende der Elektrode mit einer Energiequelle verbunden bzw. verbindbar. Die Energiequelle, alternativ auch Generator oder Strom-/Spannungsquelle, stellt die Energie zur Ausbildung eines Plasmalichtbogens bereit und ermöglicht so die Behandlung des Zielgewebes.

Der Aufsatz kann vorteilhafterweise des Weiteren einen Bereich/Abschnitt aufweisen, der dazu ausgelegt/vorgesehen ist, auf dem Zielgewebe/Körpergewebe eines Patienten aufzuliegen. Dieser Bereich kann also mit dem Zielgewebe in Anlage kommen und eine Behandlungsöffnung bzw. ein Behandlungsfenster aufweisen, die/das eine Fläche bestimmter Größe entsprechend des beabsichtigten Behandlungsbereichs definiert. Das zu behandelnde Zielgewebe befindet sich daher ausschließlich im Bereich der Öffnung/des Fensters, insbesondere dann, wenn Öffnung/Fenster auf dem Zielgewebe aufliegen. In anderen Worten ausgedrückt, ist es die Funktion der Öffnung/des Fensters, bevorzugt das zur Behandlung freigegebene Zielgewebe zu exponieren.

Dies eröffnet die grundsätzliche Möglichkeit, durch Austausch/Auswahl der Aufsätze mit unterschiedlichen Öffnungsgrößen und/oder Raumvolumen das in die Zuführeinrichtung eingeführte chirurgische Instrument für unterschiedliche Behandlungen/Zielgewebe zu optimieren. Vorzugsweise ist dieser Kontakt-/Anlagebereich des Aufsatzes, der die Öffnung/das Fenster enthält, plan ausgestaltet.

Die Montageeinrichtung/Adapter (oder Adapterabschnitt) des erfindungsgemäßen Aufsatzes ist vorzugsweise nach Art einer Hülse, Schlauch oder Tülle (oder auch eines einfachen Klebebands) ausgeformt und hat proximal eine Anschlussöffnung, die dafür angepasst ist, um an das Endoskop angeschlossen, vorzugsweise aufgesteckt/gestülpt werden zu können. Eine, durch den Mittelpunkt dieser Anschlussöffnung führende Senkrechte zur Anschlussöffnungsebene (entspricht in etwa der Tüllenmittelachse) stellt dabei eine Längsachse des gesamten Endoskopaufsatzes dar. Diese Längsachse kann axial oder parallel zur der Längsachse des Endoskops sein.

Gemäß einem bevorzugten Ausführungsbeispiel ist nunmehr die Behandlungsöffnung/das Behandlungsfenster in einem Winkel ungleich 90° und ungleich 0°, vorzugsweise zwischen 30° - 60° geneigt zur Längsachse ausgerichtet. D. h., der beabsichtigte Kontaktbereich des Endoskopaufsatzes mit dem Zielgewebe ist nicht senkrecht zur Instrumenten-/Endoskopachse sondern in einem bestimmten, vorzugsweise spitzen Neigungswinkel zur Instrumenten-/Endoskopachse in Richtung distal ausgerichtet. Vorzugsweise ist der Neigungswinkel so gewählt, dass eine in einem Funktionskanal des Endoskops untergebrachte Optik oder dergleichen Bildaufnahmegerät der Zuführeinrichtung/des Endoskops eine möglichst freie, unverdeckte Sicht auf das durch die Behandlungsöffnung/Fenster exponierte Zielgewebe hat, ohne von der in das umschlossene Raumvolumen ragenden Elektrode eingeschränkt zu werden.

Das distale Ende der Elektrode weist einen vorbestimmten Mindestabstand zu dem nächstliegenden Punkt innerhalb der genannten Fläche, vorzugsweise dem Mittelpunkt und somit auch gleichzeitig einen vorbestimmten Mindestabstand zu dem sich unter der Behandlungsöffnung befindlichen Zielgewebe auf, der durch die Aufsatzform fest definiert ist.

Liegt also der Bereich des Aufsatzes mit der Behandlungsöffnung auf dem Zielgewebe auf, kann sich der behandelnde Chirurg sicher sein, dass er, solange der Aufsatz Kontakt mit dem Zielgewebe hat, den korrekten Behandlungsabstand bzw. einen bevorzugten Mindestabstand des distalen Endes der Elektrode vom Zielgewebe einhält. Damit wird bei vorgegebener Energiestärke eine gleichbleibende Behandlungstiefe bzw. Behandlungsintensität des entstehenden Lichtbogens erreicht.

Des Weiteren besitzt das Material des Aufsatzes vorzugsweise elektrisch isolierende Eigenschaften, da es bevorzugt zumindest teilweise aus einem Nichtleiter wie Kunststoff oder Keramik besteht. Der Lichtbogen wird sich daher immer nur zwischen der Elektrode und dem im Bereich der Öffnung/des Fensters liegenden Zielgewebes ausbilden. Die fehlerhafte Ausbildung eines Lichtbogens (parasitärer Lichtbogen) zwischen Elektrode und einem nicht zu behandelnden Gewebebereich kann nicht mehr auftreten. D. h. durch die bevorzugt kappenförmige Gestalt des Aufsatzes wird garantiert, dass nur das Behandlungsgewebe durch die Behandlungsöffnung hindurch mit dem Lichtbogen beaufschlagt wird, wohingegen benachbartes Gewebe durch die elektrisch isolierende Aufsatzwand abgeschirmt ist. Das gibt dem behandelnden Chirurgen größere Sicherheit bei der Nutzung der plasmachirurgischen Geräte.

In einer weiteren Ausführungsform kann das proximale Ende der Elektrode mit einem ersten Ende eines Widerstandselements unmittelbar verbunden sein. Diese Verbindung kann eine geeignete elektrische Verbindung wie beispielsweise eine Schweißverbindung, eine Crimpverbindung, eine Lötverbindung oder ein elektrischer Steckkontakt sein.

Das zweite Ende des Widerstandselements kann über einen Leiter, der vorzugsweise in einem Katheter verlegt ist, mit einer Energieversorgung verbunden sein. Zu diesem Zweck kann das zweite Ende des Widerstandselements über eine geeignete elektrische Verbindung mit dem elektrischen Leiter im Katheter verbunden/verbindbar sein. Es kann dies, beispielsweise, eine Steckverbindung, eine Schweißverbindung, eine Schraubverbindung, eine Crimpverbindung oder eine Lötverbindung sein. Eine lösbare elektrische Verbindung vom distalen Ende des Katheters zum zweiten Ende des Widerstandselementes oder auch unter Umgehung des Widerstandselementes direkt zum proximalen Ende der Elektrode beispielsweise mittels eines geeigneten elektrischen Steckverbinders oder andere lösbare elektrische Verbindungsmittel, gewährleistet eine besondere Flexibilität beim Wechsel der Elektrode und/oder des Widerstandes.

Das Widerstandselement selbst kann durch ein oder mehrere diskrete Bauelemente gebildet sein. Die Bauelemente können auf einer kleinen Platine zu einem Elektronikmodul zusammengefasst sein. Auf dieser Platine können die Bauteile in einer parallel und/oder Reihenschaltung verschaltet sein, je nachdem, welcher Widerstandswert mit den Bauelementen erreicht werden soll.

Zur Strombegrenzung des Lichtbogens kann das Widerstandselement ein ohmscher Widerstand oder ein komplexer Widerstand wie ein Kondensator sein. Vorzugsweise werden im Handel erhältliche Kondensatoren zum Aufbau des Widerstandselementes eingesetzt.

Das Widerstandselement kann innerhalb des Aufsatzes und vorteilhafterweise innerhalb des Volumens des Aufsatzes, angeordnet sein. Auch eine spezielle Konstruktion, bei der das Widerstandselement beispielsweise der inneren Form des Aufsatzes nachgebildet ist, ist denkbar. Alternativ kann das Widerstandselement ganz vom Material der Kappe umschlossen, mithin also integraler Bestandteil der Kappe/des Aufsatzes sein.

Das Widerstandselement kann dann so in den Aufsatz integriert werden, dass bei Einsatz einer Endoskopkamera, vorteilhafterweise der Sichtbereich der Kamera nicht durch das Widerstandselement beeinträchtigt wird. Die Integration des Widerstandselementes in die Kontaktmittel zwischen Katheter und Widerstandselement selbst, ist bei Widerstandselementen mit geeigneten elektrischen Eigenschaften und entsprechender Größe möglich.

In einer weiteren, bevorzugten Ausführungsform, kann das Endoskop, für das der Aufsatz gedacht ist, einen oder mehrere Funktionskanäle aufweisen. Funktionskanäle sind röhrenförmige Hohlräume in einem Endoskop, die es erlauben, von außerhalb des Körpers für den Durchmesser des Endoskopkanals (Arbeitskanal) angepasste Werkzeuge bis an die Eingriffsstelle im Körper eines Patienten zu bringen.

Zur Beobachtung des Arbeitsgebietes bzw. des zu behandelnden Zielgewebes kann beispielsweise eine Endoskopkamera in einem weiteren Funktionskanal des Endoskops angeordnet werden. Alternativ kann die Kamera und gegebenenfalls Beleuchtung fester Bestandteil des Endoskops sein.

Im Funktions- oder Arbeitskanal des Endoskops kann ein Katheter angeordnet/eingeführt sein/werden, wobei innerhalb des Katheters ein elektrischer Leiter angeordnet sein kann. Das distale Ende des Katheters kann elektrische Kontakteinrichtungen aufweisen, die so ausgebildet und dazu geeignet sind, an ein Ende des Widerstandselementes oder direkt an das proximale Ende der Elektrode angeschlossen zu werden. Widerstandselement und/oder Elektrode weisen dazu korrespondierende elektrische Kontakteinrichtungen/Anschlüsse auf.

Im Falle, dass das Widerstandselement zur Begrenzung des Behandlungsstroms schon im Generator selbst mit eingebaut ist, kann an die Kontaktmittel am distalen Ende des Katheters auch das proximale Ende der im Aufsatz angeordneten Elektrode direkt angeschlossen werden.

Der Katheter kann, zusätzlich zu dem erwähnten elektrischen Leiter, ein Lumen aufweisen, wodurch ein Gas, vorzugsweise ein Edelgas, an den Behandlungsort geleitet werden kann. Das Gas füllt während der Behandlung das durch die Kappe bzw. den Aufsatz umschlossene Innenvolumen des Aufsatzes/der Kappe. Dadurch, dass der Aufsatz/die Kappe eine Behandlungsöffnung lediglich zum Zielgewebe aufweist, bleibt das Gas besser im Bereich der Behandlungsstelle konzentriert. Da immer frisches Gas in den Aufsatzraum/die Kappe nachströmt, wird der eventuell beim Behandeln mit dem Plasmalichtbogen entstehende Rauch/Dampf gezielt über die Öffnung von der Behandlungsstelle weggeleitet. Damit wird er vorteilhafter Weise auch aus dem Sichtbereich bzw. dem Bereich der von einer in einem anderen Arbeitskanal des Endoskops platzierten Endoskopkamera beobachteten Bereich entfernt.

In weiter vorteilhafter Weise kann der Aufsatz des chirurgischen Instruments/ Endoskops im Bereich der Behandlungsöffnung Markierungen aufweisen. Diese Markierungen können beispielsweise eine Skala bilden, die so angeordnet ist, dass sie einen Rückschluß auf die Größenverhältnisse des in der Behandlungsöffnung/im Behandlungsfenster sichtbaren und zu behandelnden Zielgewebes zulässt.

Diese Markierungen/diese Skala können über eine Optik/Kamera, die sich auch zusätzlich in einem anderen Endoskopkanal/Funktionskanal befinden kann, auf dem Kamerabild bzw. auf einem externen Monitor sichtbar sein.

Die Markierungen/die Skala können/kann im Material des Aufsatzes eingelassen sein oder eine reliefartige Struktur aufweisen. Sie können auch auf dem Material des Aufsatzes aufgedruckt sein.

In einer weiteren bevorzugten Ausführungsform kann der Aufsatz einteilig aus der Kappe und der Montageeinrichtung/Adapter ausgebildet sein. Bei einer einteiligen Ausbildung kann der Teil des Aufsatzes, der mit dem Endoskop verbunden/verbindbar ist, aus einem elastischen Material bestehen.

Der kappenförmige Teil des Aufsatzes kann aus einem vergleichsweise starren und elektrisch isolierenden Material bestehen. Vorteilhafterweise kann das Material des Aufsatzes ganz oder teilweise aus einem transluzenten Material bestehen.

Ein einteiliger Aufsatz mit einer verwindungssteifen Kappe und einem gummielastischen Ende/Adapter zur Anbringung an ein Endoskop kann beispielsweise mit einer 2-K Technik im Spritzgussverfahren hergestellt sein.

Eine zweiteilige Ausbildung des Aufsatzes ist aber auch möglich und auch bevorzugt. Wenn der Aufsatz zweiteilig ausgebildet ist, sind der kappenförmige Teil/Kappe und die Montageeinrichtung/Adapter, zwei einzelne Elemente vorzugsweise aus unterschiedlichen Materialien mit unterschiedlichen mechanischen Eigenschaften.

Die Montageeinrichtung/der Adapter dient dann dazu, mit seinem proximalen Ende am distalen Ende der Zuführeinrichtung/des Endoskops (durch Aufstecken) befestigt zu werden. Aus diesem Grund ist es vorteilhaft, wenn die Montageeinrichtung/der Adapter, welche auch als Tülle oder Hülse bezeichnet werden kann, aus einem gummielastischen Material oder einem einfachen Klebeband besteht. Damit kann sie auf Endoskope verschiedener Durchmesser aufgesteckt oder befestigt werden. Die Tülle kann auch aus einem nichtelastischen Material bestehen. In diesem Falle kann die Tülle entsprechend ihrer Abmessungen nur auf dem für den Durchmesser der Tülle passenden Endoskoptyp befestigt werden.

An einem anderen, distalen Ende kann die Tülle dazu ausgebildet sein, mit einer Seite der Kappe verbunden zu werden. In dem Bereich, in dem Tülle und Kappe miteinander verbunden werden, können sowohl Tülle als auch Kappe mit miteinander korrespondierenden Verbindungsmitteln ausgestattet sein, wie beispielsweise einem Bajonettverschluss oder einer Verschraubung. Auch eine Klebverbindung ist denkbar. Diese Verbindungen zwischen der Tülle und der Kappe erlauben es, verschiedene Kappen für ein und dasselbe Endoskop zu verwenden, da die Tülle am Endoskop verbleiben kann. Alternativ kann der gesamte Aufsatz einschließlich Tülle und Kappe gewechselt werden.

Durch den Wechsel der Kappe/des Aufsatzes kann, in dem Fall wenn Widerstandselement und Elektrode Teil der Kappe/des Aufsatzes sind, Einfluss auf die Behandlungstiefe genommen werden, die von den Werten des Widerstandselementes abhängig sind.

Weiter vorteilhaft kann der Aufsatz zur Überwachung der thermischen Verhältnisse, eine Temperaturmessvorrichtung wie etwa einen NTC Widerstand oder eine sonstige, zur Messung einer absoluten Temperatur oder einer Temperaturänderung geeignete Einrichtung beinhalten.

Zusätzlich kann diese Einrichtung so gestaltet sein, dass die im Zuge der Temperaturmessung erhaltenen Werte übertragen werden. Die Information über die Temperatur im Aufsatz kann beispielsweise über den elektrischen Leiter des Katheters übertragen und als elektronisch verarbeitbare Information ausgegeben werden, z.B. mittels eines Multiplexverfahrens oder dergleichen. Eine drahtlose Übertragung ist mittels einer geeigneten Einrichtung im Aufsatz oder in dem Endoskop ebenso möglich. Zur Visualisierung kann der Temperaturwert beispielsweise in das Kamerabild der Endoskopkamera eingeblendet werden. Eine externe Anzeige an einem dafür vorgesehenen Gerät ist ebenso denkbar.

### Figurenbeschreibung

Die Erfindung wird nachstehend anhand eines besonderen Ausführungsbeispiels unter Bezugnahme auf die begleitenden Figuren näher erläutert.
Fig. 1 zeigt die einzelnen Elemente eines plasmachirurgischen Instruments gemäß einem bevorzugten Ausführungsbeispiel der Erfindung insbesondere einen Aufsatz, bestehend aus einem kappenförmigem ersten Teil und einer Tülle bzw. Montageeinrichtung/Adapter als zweitem Teil zur wahlweisen Befestigung an einer Zuführeinrichtung/einem Endoskop sowie die im Aufsatz liegenden Teile wie Elektrode, Widerstandselement und Anschlussbuchse, wobei der Katheter/elektrisches Kabel des Instruments nur angedeutet ist,
Fig. 2 zeigt den Aufsatz des plasmachirurgischen Instruments mit der Montageeinrichtung/Adapter am Endoskop und der im Innenbereich des Aufsatzes liegenden Elektrode sowie einer Behandlungsöffnung/Fenster im Aufsatz,
Fig. 3 zeigt die Montageeinrichtung/Adapter des Aufsatzes bei einer zweiteiligen Ausführung des Aufsatzes aus Sichtrichtung vom proximalen Ende des kappenförmigen Teils,
Fig. 4 zeigt eine Innenansicht des Aufsatzes mit der Behandlungsöffnung und insbesondere um die Behandlungsöffnung herum angeordneten Skalenmarkierungen/ Markierungen,
Fig.5 zeigt eine Ausführungsform der Elektrode,
Fig.6 zeigt eine vergrößerte Ansicht der Anschlussbuchse zur Kontaktierung der Kontaktmittel (elektrische Leiter/Kabel) eines in dem Endoskop verlegten/eingeführten Katheters und
Fig. 7 zeigt eine Steckerkonstruktion am distalen Ende der elektrischen Leitung des Katheters zum Anschluss an der Anschlussbuchse.

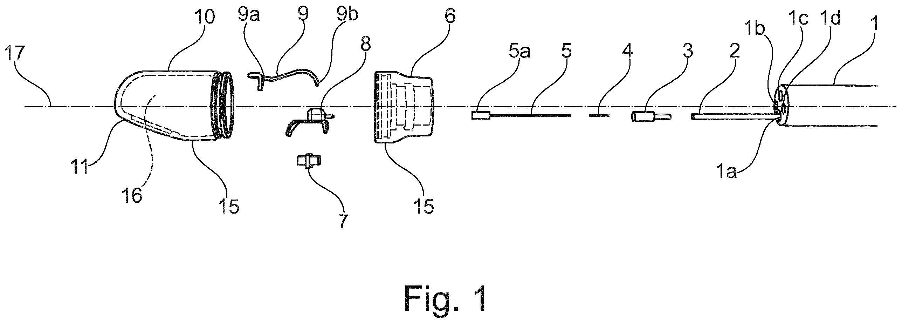

In Fig. 1 sind die wesentlichen Komponenten eines vorzugsweise minimalinvasiven plasmachirurgischen Gerätes/Instruments gemäß einem bevorzugten Ausführungsbeispiel der Erfindung in Explosionszeichnung zu erkennen. Das plasmachirurgische Gerät/Instrument ist gemäß dem vorliegenden Ausführungsbeispiel zur Einführung in den Arbeitskanals einer Zuführeinrichtung vorzugsweise in Form eines Endoskops 1 vorgesehen, die, wie üblich, mit mehreren (vorliegend vier) Funktions-/Arbeitskanälen 1a bis1d ausgebildet ist, wobei insbesondere das Endoskop in der Regel nur einen Arbeitskanal für das Einführen eines medizinischen Werkzeugs aufweist und die übrigen Kanäle Funktionskanäle beispielsweise für Beleuchtung, Optik, Absaugung, Spülung, etc. vorgesehen sind.

Ein Katheter 2 (Werkzeug) des erfindungsgemäßen chirurgischen Instruments ist im Arbeitskanal 1a des Endoskops 1 verlegt bzw. in diesen eingeführt/einführbar. Der Katheter 2 enthält unter anderem elektrische Kontaktmittel (Kabel, Drähte, Kabelanschlüsse) 3-5a, die zur Zuführung von Energie von einem Generator (nicht gezeigt) bis zu einer Elektrode 9 am distalen Ende des chirurgischen Instruments (des Endoskops 1) notwendig sind. Eine schlauch- oder hülsenförmige Montageeinrichtung/Adapter 6 ist zu erkennen, welche eine Axialseite mit geringerem und eine Axialseite mit einem größeren Durchmesser aufweist. Die Seite mit kleinerem Durchmesser, die in Richtung hin zum distalen Ende des Endoskops 1 zeigt, bildet jene Seite, mit der die Montageeinrichtung/Adapter 6, oder auch Tülle, am Endoskop 1 insbesondere an dessen Endoskopkopf befestigt wird/ist. Hierfür bildet die Seite mit kleinem Durchmesser einen innenwandig im Wesentlichen glatten, vorzugsweise elastischen Schlauchabschnitt, der auf das Endoskop 1 aufgezogen/übergestülpt werden kann, um dort federelastisch/reibschlüssig zu haften. Die Seite mit großem Durchmesser, welche ebenfalls einen elastischen Schlauchabschnitt darstellt, hat indessen einen innenseitig umlaufenden Ringvorsprung oder Wulst.

Eine Applikationskappe 10 ist an der distalen Axialseite der Tülle 6 mit großem Durchmesser montiert oder montierbar. Die Applikationskappe 10 ist hierfür in ihrer äußeren Gestalt einem Fingerhut oder Napf nachempfunden mit einem abgerundeten distalen Endabschnitt und einem hülsenförmigen proximalen Endabschnitt, an dessen Außenumfang eine Rastnut ausgebildet ist. Zur Montage der Applikationskappe 10 an der Tülle 6 wird diese über den hülsenförmigen proximalen Kappenendabschnitt gezogen, wodurch die tüllenseitige Wulst in der kappenseitigen Rastnut formschlüssig verrastet. Auf diese Weise kann die Applikationskappe 10 unabhängig von deren Größe, Durchmesser und/oder Form über die elastische Tülle 6 mit einem (jedem) Standardendoskop mechanisch konnektiert werden.

An dieser Stelle sein nochmals hervorgehoben, dass die Zuführeinrichtung (Endoskop) 1 nicht Bestandteil des erfindungsgemäßen chirurgischen Instruments ist, sondern lediglich als Einführmittel für das chirurgische Instrument in den Innenraum eines Patienten dient. Das chirurgische Instrument selbst besteht aus dem Katheter (mit innen liegendem Kabel), der daran anschließbaren oder angeschlossenen Elektrode (ggf. mit unmittelbar vorgeschaltetem Widerstand) und einem Abstandshalter bestehend aus der Applikationskappe 10 sowie dem Montageadapter 6.

Die Applikationskappe 10 und die Tülle 6 bilden zusammen den Abstandshalter/Endoskopaufsatz 15, der in diesem bevorzugten Fall aus den zwei genannten separaten Teilen 6, 10 besteht. Auf diese Weise kann die Tülle 6 z. B. insgesamt aus einem elastischen Material und die Kappe 10 aus einem starren/steifen Material bestehen. Alternativ kann der Endoskopaufsatz 15 mit beiden Funktionsabschnitten aber auch stoffeinstückig ausgebildet sein.

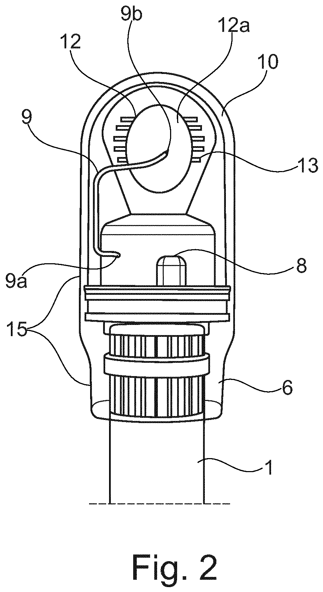

Die Applikationskappe 10 weist gemäß der Fig. 1 und 2 einen vorzugsweise abgeflachten/planen (Auflage-) Bereich 11 auf, der dafür vorgesehen ist, um mit einem Zielgewebe eines Patienten in Berührung/Anlage zu kommen. Unter der Annahme, dass die hülsenförmige Tülle 6 eine Mittel-/Axialachse 17 hat, welche gleichzeitig auch die Längsachse der Applikationskappe 10 bildet, liegt dieser plane Bereich 11 außermittig sowie in einem spitzen Winkel in Richtung distal geneigt zu der Längsachse. In dem im Wesentlichen planen Bereich 11 ist ferner eine Behandlungsöffnung/Fenster 12 ausgebildet, über welche ein von der Applikationskappe 10 umschlossenes Raumvolumen 16 zur Atmosphäre Fluidkontakt hat.

Die Elektrode 9, eine Anschlussbuchse (Kontaktmittel) 8 und eine Elektronik (Widerstandselement) 7 sind ebenfalls in der Fig. 1 zu erkennen. Diese sind innerhalb des Raumvolumens 16 des Aufsatzes 15 bzw. der Applikationskappe 10 angeordnet und mit einer elektrischen Leitung 4, 5 innerhalb des Katheters 2 elektrisch verbunden/verbindbar. Dabei sei darauf hingewiesen, dass die Funktionskanäle 1b-1d im Endoskop 1 in der Regel außermittig um den Arbeitskanal 1a angeordnet sind. Daher positioniert sich eine Optik des Endoskops 1 ebenfalls außermittig. Da aber das Behandlungsfenster 12 ebenfalls außermittig angeordnet ist, kann der Aufsatz 15 bezüglich der Optik so am Endoskopkopf rotiert werden, dass die Sichtlinie zwischen Optik und Behandlungsfenster 12 frei ist und nicht etwa von der in das Raumvolumen 16 ragenden Elektrode 9 versperrt wird.

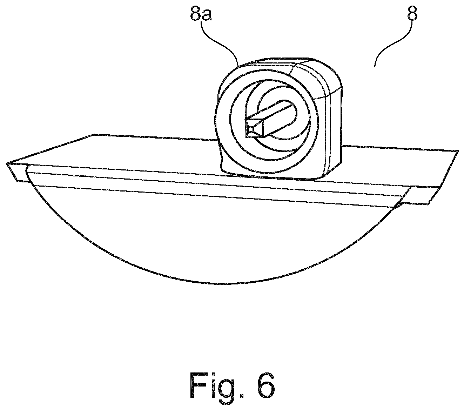

Insbesondere ist innerhalb der Tülle 6 eine Art Montagesockel (nicht weiter dargestellt) vorgesehen, an welchem eine klammerartige Halterung fixierbar ist, wie diese insbesondere in der Fig. 6 in Vergrößerung dargestellt ist. An der Halterung sind die Anschlussbuchse 8 sowie das Widerstandselement 7 montiert, welche somit in der Tülle 6 fixierbar sind. Das Widerstandselement 7 ist mit der Anschlussbuchse 8 elektrisch verbunden.

Fig. 2 zeigt eine Seitenansicht auf das Endoskop 1 und insbesondere dessen Endoskopkopf, an welchem die Tülle 6 und über diese die Applikationskappe 10 angebracht sind. Ein proximales Ende 9a der Elektrode 9 ist mit der Anschlussbuchse 8 verbunden und über die Halterung in der Tülle 6 fixiert. Eine distale Spitze 9b der Elektrode 9 ragt dabei in das Raumvolumen 16 des Aufsatzes 15/der Applikationskappe 10. Da die Anbringung der Buchse 8 und damit auch die der Elektrode 9 relativ zum Aufsatz 15 fix ist, ergibt sich somit ein konstruktiver Mindestabstand zwischen der Elektrodenspitze 9b und der Behandlungsöffnung 12.

Die Montageeinrichtung/Adapter/Tülle 6 ist, wie bereits ausgeführt wurde, aus einem gummielastischen Material, beispielsweise aus Silikongummi, gebildet, welches eine flexible Kopplung mit Endoskopen 1 unterschiedlicher Durchmesser ermöglicht. Der kappenförmige Teil 10, ist in dieser Ausführungsform aus einem elektrisch isolierenden Material mit einer vorbestimmten Steifigkeit (höher als die Tülle 6) beispielsweise ein Kunststoff oder Keramik gefertigt, das weiter vorzugsweise lichtdurchlässig oder sogar durchsichtig ist.

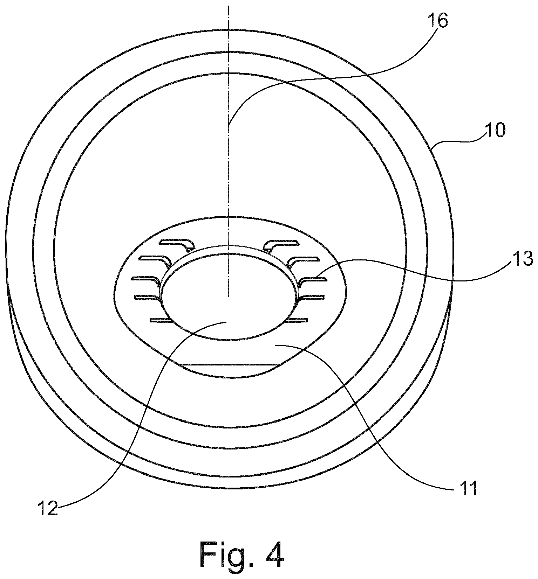

Um die Behandlungsöffnung 12 herum sind insbesondere mit Verweis auf die Fig. 4 Markierungen/Skaleneinteilungen 13 zu erkennen. Diese Markierungen 13 helfen dem Benutzer während des Eingriffs bei der Orientierung hinsichtlich der Größe/Dimension des zu behandelnden Zielgewebes, wenn nämlich die Applikationskappe 10, mit dem planen Bereich in dem sich die Behandlungsöffnung 12 befindet, auf dem Zielgewebe aufliegt. Die Markierungen 13 können alternativ auch als Skala oder Skalenmarkierungen bezeichnet werden.

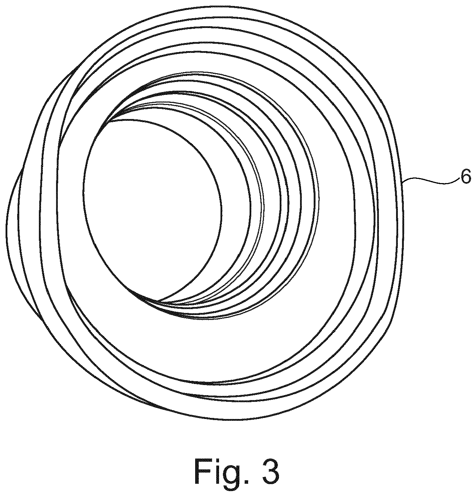

Fig. 3 zeigt eine perspektivische Vorderansicht der Montageeinrichtung/Adapter (Tülle) 6. Im Vordergrund ist das großdurchmessrige distale Ende der Montageeinrichtung/Adapter 6 mit Innen-Umfangswulst zu erkennen, welches mit dem proximalen Ende des kappenförmigen Teils (Applikationskappe) 10 gekoppelt wird. Diese Montageeinrichtung/Adapter/Tülle 6 kann somit wahlweise auf der Applikationskappe 10 oder auf dem Endoskop 1 verbleiben, wenn der Aufsatz 15 ausgetauscht werden soll. Durch diese Konstruktion wird somit ermöglicht, auf ein und dasselbe Endoskop 1 verschiedene Aufsätze 15 mit unterschiedlichen Elektroden 9 bzw. Widerstandselementen 7, unterschiedlichen Raumvolumen 16 und/oder unterschiedlich dimensionierten Behandlungsöffnungen 12 zu montieren. Da sich die Widerstandselemente 7 innerhalb des Aufsatzes 15 befinden, kann man mit einem Wechsel des Aufsatzes 15 unterschiedliche Widerstandswerte und damit unterschiedliche Behandlungstiefen auswählen.

Die Innenansicht des kappenförmigen Teils/Kappe 10 in Fig. 4 zeigt die Behandlungsöffnung bzw. Fenster 12 und den planen Bereich 11, der idealerweise bei der Behandlung auf dem Zielgewebe aufliegt. Um das Fenster 12 herum angeordnet befinden sich die vorstehend genannten Markierungen/Skalenteilungen 13. Die Größe dieser Markierungen/Skalenteilungen 13 ist dem behandelnden Arzt bekannt bzw. ist konstruktiv vorbestimmt. Beispielsweise kann der Abstand zwischen zwei Skalenteilen einen bestimmten Wert in mm aufweisen. Daher kann der behandelnde Arzt sofort auf einem Bild, welches beispielsweise von einer Kamera, insbesondere einer Endoskopkamera, ausgegeben werden kann, eine genaue Größenabschätzung von Objekten abgeben, die sich innerhalb des Fensters 12 befinden.

Durch den elektrisch isolierenden Effekt von Kunststoff oder Keramik zumindest für die Applikationskappe 10 ergibt sich eine vorteilhafte elektrische Isolierung des Innenraums 16 des Aufsatzes 15 gegenüber der Umgebung. Der Plasmalichtbogen kann daher seine Wirkung nur auf das Gewebe entfalten, welches innerhalb des Bereichs der Behandlungsöffnung 12 (Öffnungsfläche 12a) sichtbar ist, wenn der plane Bereich 11 auf dem Zielgewebe aufliegt.

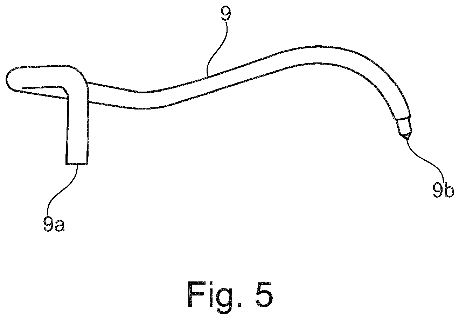

Die Elektrode 9 in Fig. 5 hat einen vorzugsweise mehrfach gekrümmten Längsverlauf, um die Sichtlinie zwischen der Endoskopoptik und der Behandlungsöffnung möglichst zu umgehen und weist an ihrem distalen Ende 9b eine Spitze auf. Das proximale Ende 9a der Elektrode 9 ist mit einer Seite des Widerstandselementes 7 (hier nicht zu sehen) unmittelbar elektrisch verbunden. Die Lage der Elektrode 9 im Aufsatz 15 ist fix relativ zur Behandlungsöffnung 12. Der Lichtbogen zündet zwischen der Elektrodenspitze 9b der Elektrode 9 und dem Zielgewebe, das durch das Behandlungsfenster 12 sichtbar ist.

Die Anschlussbuchse 8 in Fig. 6 verbindet den elektrischen Leiter 4, 5 des Katheters 2 mit einer Seite des hier nicht gezeigten Widerstandselementes 7. Hierfür hat die Anschlussbuchse ein steckdosenförmiges Gehäuse, in der ein in das Gehäuse vorragender Kontaktstift 8a angeordnet ist. Der Kontaktstift 8a ist elektrisch an das Widerstandselement 7 angeschlossen, das wiederum an die Elektrode 9 unmittelbar angeschlossen ist. Das Buchsengehäuse ist gemäß der Fig. 6 an der klammerartigen Halterung fixiert, kann aber auch einstückig mit dieser ausgebildet sein.

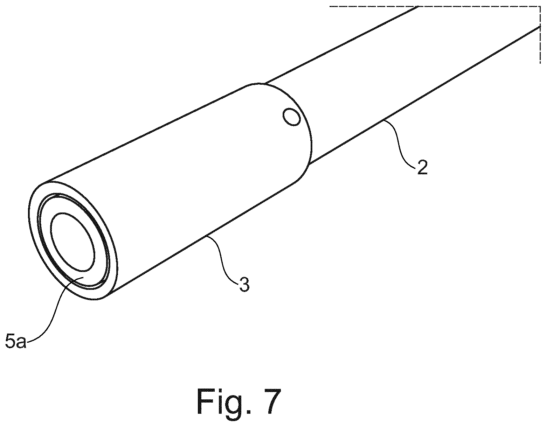

Fig. 7 zeigt den Anschlussstecker auf Seiten der elektrischen Leitung des Katheters 2 in Vergrößerung.

Demzufolge besteht der Katheter 2 aus einem rohrförmigen Mantel aus elektrisch isolierenden Material wie einem Kunststoff, auf den endseitig eine Steckerhülse 3 aufgesteckt ist. Innerhalb des Mantels befindet sich der elektrische Leiter oder Draht 4, 5 der endseitig eine Aufnahmelasche 5a hat, die für das Aufstecken auf den Kontaktstift 8a ausgebildet ist. Die Aufnahmelasche 5a wird dabei umfangsseitig von der Steckerhülse 3 kontaktlos umgeben.

Um die Elektrode 9 an die elektrische Energiequelle anzuschließen, wird die Steckerhülse 3 reib- oder formschlüssig in die Anschlussbuchse 8 bzw. deren Gehäuse eingesteckt. Dabei wird die Aufnahmelasche 5a automatisch auf den Kontaktstift 8a aufgesteckt. Schließlich kann die Tülle 6 unter Zurückziehen des Katheters 2 auf das Endoskop 1 aufgestülpt und die Applikationskappe 10 an der Tülle 6 verrastet werden.

Die mechanische Verbindung zwischen der Anschlussbuchse und dem Stecker 5a kann als reine Steckverbindung ausgebildet sein, wie die vorstehend beschrieben wurde. Sie kann aber auch als eine Schraub- oder Bajonettverbindung ausgelegt sein.

Offenbart wird zusammenfassend ein Abstandshalter in Form eines Aufsatzes 15 zur Montage an einem Endoskop 1, wobei der Aufsatz 15 kappenförmig ausgebildet ist und ein Raumvolumen umschließt sowie aus einem isolierenden Material besteht. Der Aufsatz 15 ist weiterhin mit einer Montageeinrichtung/Adapter 6 ausgerüstet, die so ausgebildet ist, um an ein distales Ende des Endoskops 1 angekoppelt zu werden. Der Aufsatz 15 hat einen Bereich 11, der ausgelegt ist, mit einem Zielgewebe in Kontakt zu stehen und der weiter eine Öffnung 12 definiert, die eine Fläche 12a umschließt. Innerhalb des Raumvolumens ist eine Elektrode 9 mit einem distalen 9b und einem proximalen 9a Ende angeordnet, wobei das distale 9b Ende der Elektrode 9 einen vorbestimmten Mindestabstand zum nächstliegenden Punkt der Fläche 12a aufweist. Der nächstliegende Punkt ist dabei jener Punkt der Fläche 12a durch den eine Senkrechte zur Fläche geht, welche das distale Ende der Elektrode 9 schneidet.

**Bezugszeichenliste:**

| | |
|---|---|
| Endoskop | 1 |
| Arbeits-/Funktionskanäle des Endoskops | 1a-d |
| Katheter | 2 |
| Steckerhülse | 3 |
| elektrischer Leiter | 4, 5 |
| Kontaktlasche | 5a |
| Montageeinrichtung/Adapter, Tülle Elektronik/Widerstandselement | 6 |
| Buchse/Anschlussbuchse | 8 |
| Elektrode | 9 |
| Elektrodenspitze (distales Ende) | 9a |
| Elektrode (proximales Ende) | 9b |
| Kappe | 10 |
| Gewebe-Anlagebereich | 11 |
| Öffnung/Fenster der Kappe | 12 |
| Fläche | 12a |
| Markierungen | 13 |
| Aufsatz | 15 |
| Raumvolumen | 16 |
| Längsachse | 17 |

## Patentansprüche

1. Plasmachirurgisches Instrument der minimalinvasiven Bauart, wobei am distalen Ende des Instruments eine Elektrode (9) vorgesehen ist, die dafür angepasst ist, bei Zufuhr von elektrischer Energie in einer bestimmten Gasumgebung sowie bei einem bestimmten Abstand zu einem Zielgewebe einen Lichtbogen zu erzeugen, mit einem einen Abstandshalter bildendenAufsatz (15) aus einem elektrisch isolierenden Material, der an seinem distalen Abschnitt ein Raumvolumen (16) umschließt und an seinem proximalen Abschnitt eine Montageeinrichtung oder einen Adapter (6) aufweist, die/der dafür ausgebildet ist, um an ein distales Ende einer endoskopischen Zuführvorrichtung (1) für das plasmachirurgische Instruments angekoppelt zu werden, wobei der Aufsatz (15) einen Wandbereich (11) aufweist, der ausgelegt ist, mit dem Zielgewebe in Kontakt zu kommen, um einen Mindestabstand des distalen Endes (9b) der Elektrode (9) vom Zielgewebe einzuhalten, wobei die Montageeinrichtung oder der Adapter (6) proximal eine Anschlussöffnung hat, die dafür ausgebildet ist, auf die endoskopische Zuführeinrichtung (1) aufgesteckt oder aufgestülpt zu werden und der Aufsatz (15) an seinem das Raumvolumen (16) umschließenden distalen Abschnitt eine Applikationskappe (10) ausbildet, welche den mit dem Zielgewebe in Kontakt kommenden Wandbereich (11) enthält, in welchem eine Behandlungsöffnung (12) mit definierter Öffnungsfläche (12a) ausgebildet ist, die so positioniert und/oder ausgerichtet ist, dass die in das Raumvolumen (16) ragende Elektrode (9) und insbesondere deren distales Ende (9b) einen vorbestimmten Mindestabstand zum nächstliegenden Punkt, vorzugsweise Mittelpunkt, der Öffnungsfläche (12a) für die Erzeugung des Lichtbogens hat, wobei die Behandlungsöffnung (12) dafür vorgesehen und ausgebildet ist, auf dem Zielgewebe aufzuliegen.

2. Plasmachirurgisches Instrument nach Anspruch 1, **dadurch gekennzeichnet, dass**
der Aufsatz (15) zweiteilig ausgebildet ist und aus der vorzugsweise fingerhutförmigen Applikationskappe (10) vorzugsweise aus einem starren Material und der Montageeinrichtung (6) besteht, die eine schlauchförmige Tülle vorzugsweise aus einem gummi-elastischen Material bildet oder aus einem Klebeband gefertigt ist.

3. Plasmachirurgisches Instrument nach Anspruch 2, **dadurch gekennzeichnet, dass** die Montageeinrichtung und vorzugsweise die Tülle (6) eine Mittelachse hat, zu der die Behandlungsöffnung (12) unter einem spitzen Winkel in Richtung distal geneigt ist.

4. Plasmachirurgisches Instrument nach Anspruch 3, **dadurch gekennzeichnet, dass** die Behandlungsöffnung (12) bezüglich der Mittelachse außermittig angeordnet ist.

5. Plasmachirurgisches Instrument nach einem der vorstehenden Ansprüche, **dadurch gekennzeichnet, dass** der Aufsatz (15) an seinem distalen Ende in axialer Richtung verrundet ist.

6. Plasmachirurgisches Instrument nach einem der vorstehenden Ansprüche, **dadurch gekennzeichnet, dass** die Elektrode (9) am Aufsatz (15) unter Beibehaltung des vorbestimmten Mindestabstands fixiert ist und über eine elektrische Anschlusseinrichtung (7, 8) innerhalb oder am Aufsatz (15) an eine elektrische Leitung (4, 5) anschließbar ist, die dafür angepasst ist, durch die Zuführeinrichtung (1) hindurchgeschoben zu werden.

7. Plasmachirurgisches Instrument nach Anspruch 6, **dadurch gekennzeichnet, dass** die elektrische Anschlusseinrichtung (7, 8) ein der Elektrode (9) unmittelbar vorgeschaltetes Widerstandselement (7) und eine dem Widerstandselement (7) unmittelbar vorgeschaltete Anschlussbuchse (8) für die elektrische Leitung (4, 5) hat.

8. Plasmachirurgisches Instrument nach Anspruch 7, **dadurch gekennzeichnet, dass**
das Widerstandselement (7) und vorzugsweise die Anschlussbuchse (8) über eine gemeinsame Halterung am Aufsatz (15) und vorzugsweise an der Applikationskappe (10) oder an der Tülle (6) montiert ist.

9. Plasmachirurgisches Instrument nach einem der vorstehenden Ansprüche, **dadurch gekennzeichnet, dass** im Bereich der Behandlungsöffnung (12) zum Zielgewebe Markierungen/Skalenteilungen/eine Skala (13) angeordnet sind/ist, zur Größenbeurteilung während einer Behandlung für Objekte, die innerhalb der Öffnung (12) am Zielgewebe sichtbar sind.

10. Plasmachirurgisches Instrument nach einem der vorhergehenden Ansprüche, **dadurch gekennzeichnet, dass**
der Aufsatz (15) eine Temperaturmessvorrichtung beinhaltet, die ausgebildet ist, eine Innentemperatur des Raumvolumens zu messen und als verarbeitbare Information auszugeben.

## Claims

1. A plasma-surgical instrument of the minimally invasive type, a distal end of the instrument being provided with an electrode (9) which, when supplied with electrical energy, is adapted to generate an electric arc in a specific gaseous atmosphere and with a specific distance to a target tissue, including an attachment (15) which forms a spacer, is made of an electrically insulating material, encloses a spatial volume (16) at its distal section and, at its proximal section, comprises a mounting device or an adapter (6) designed to be coupled to a distal end of an endoscopic feeding device (1) for the plasma-surgical instrument, the attachment (15) comprising a wall area (11) which is designed to come into contact with the target tissue so as to maintain a minimum distance of the distal end (9b) of said electrode (9) to the target tissue, wherein
the mounting device or adapter (6) at its proximal end has a connection opening configured to be attached to or put on top of an endoscopic feeding device (1), and the attachment (15), at its distal section enclosing the spatial volume (16), forms an application cap (10) which includes the wall area (11) that comes into contact with the target tissue and in which a treatment aperture (12) with a defined aperture area (12a) is formed, which is positioned and/or oriented such that the electrode (9) protruding into the spatial volume (16) and in particular its distal end (9b) has a predetermined minimum distance to the closest point, preferably the central point of the aperture area (12a), for the generation of the electric arc, the treatment aperture (12) being provided and formed to lie on the target tissue.

2. The plasma-surgical instrument according to claim 1, **characterized in that** the attachment (15) is formed in two pieces and consists of a preferably thimble-shaped application cap (10) preferably made of a rigid material, and the mounting device (6) which forms a tubular bushing preferably of an elastic rubber material or is made from an adhesive tape.

3. The plasma-surgical instrument according to claim 2, **characterized in that** the mounting device and preferably the bushing (6) has a central axis relative to which the treatment aperture (12) is inclined at an acute angle in distal direction.

4. The plasma-surgical instrument according to claim 3, **characterized in that** the treatment aperture (12) is arranged to be off-center with respect to the central axis.

5. The plasma-surgical instrument according to any of the preceding claims, **characterized in that** the attachment (15) has its distal end rounded in the axial direction.

6. The plasma-surgical instrument according to any of the preceding claims, **characterized in that** the electrode (9) is fixed to the attachment (15) while maintaining the predetermined minimum distance and can be connected, via an electrical connection means (7, 8) within or on the attachment (15), to an electrical line (4, 5) adapted to be pushed through the feeding device (1).

7. The plasma-surgical instrument according to claim 6, **characterized in that** the electrical connection means (7, 8) comprises a resistive element (7) immediately upstream of the electrode (9) and a connecting socket (8), for the electrical line (4, 5), immediately upstream of the resistive element (7).

8. The plasma-surgical instrument according to claim 7, **characterized in that** the resistive element (7) and preferably the connecting socket (8) is mounted to the attachment (15) and preferably to the application cap (10) or the bushing (6) via a common support.

9. The plasma-surgical instrument according to any of the preceding claims, **characterized in that** markings/scale divisions/a scale (13) are/is arranged in the area of the treatment aperture (12) facing the target tissue, said markings/scale divisions/scale during a treatment serving for evaluating the size of objects which are visible within the aperture (12) on the target tissue.

10. The plasma-surgical instrument according to any of the preceding claims, **characterized in that** the attachment (15) includes a temperature measuring device which is designed to measure an inside temperature of the spatial volume and to output it as processible information.

## Revendications

1. Instrument de chirurgie au plasma du type mini-invasif, dans lequel une électrode (9) est prévue à l'extrémité distale de l'instrument, qui est adaptée pour générer un arc électrique lors de l'apport d'énergie électrique dans un environnement gazeux déterminé ainsi qu'à une distance déterminée par rapport à un tissu cible, avec une coiffe (15) en un matériau électriquement isolant formant une entretoise, qui entoure au niveau de sa section distale un volume d'espace (16) et présente au niveau de sa section proximale un dispositif de montage ou un adaptateur (6), qui est réalisé pour être accouplé à une extrémité distale d'un dispositif d'alimentation endoscopique (1) pour l'instrument de chirurgie au plasma, dans lequel la coiffe (15) présente une zone de paroi (11), qui est conçue pour entrer en contact avec le tissu cible pour respecter une distance minimum de l'extrémité distale (9b) de l'électrode (9) par rapport au tissu cible, dans lequel le dispositif de montage ou l'adaptateur (6) a proximalement une ouverture de raccordement, qui est réalisée pour être enfichée ou enfoncée sur le dispositif d'alimentation endoscopique (1) et la coiffe (15) forme au niveau de sa section distale entourant le volume d'espace (16) un capot d'application (10), lequel contient la zone de paroi (11) entrant en contact avec le tissu cible, dans laquelle une ouverture de traitement (12) avec aire d'ouverture (12a) définie est réalisée qui est positionnée et/ou orientée de telle sorte que l'électrode (9) faisant saillie dans le volume d'espace (16) et en particulier son extrémité distale (9b) a une distance minimum prédéterminée par rapport au point le plus proche, de préférence point médian, de l'aire d'ouverture (12a) pour la génération de l'arc électrique, dans lequel l'ouverture de traitement (12) est prévue et réalisée pour reposer sur le tissu cible.

2. Instrument de chirurgie au plasma selon la revendication 1, **caractérisé en ce que**
la coiffe (15) est réalisée en deux parties et est constituée du capot d'application (10) de préférence en forme de dé à coudre de préférence en un matériau rigide et du dispositif de montage (6), qui forme un embout tubulaire de préférence en un matériau caoutchouc-élastique ou est réalisé en une bande adhésive.

3. Instrument de chirurgie au plasma selon la revendication 2, **caractérisé en ce que** le dispositif de montage et de préférence l'embout (6) a un axe médian, vers lequel l'ouverture de traitement (12) est inclinée dans la direction distale en formant un angle aigu.

4. Instrument de chirurgie au plasma selon la revendication 3, **caractérisé en ce que** l'ouverture de traitement (12) est agencée excentriquement par rapport à l'axe médian.

5. Instrument de chirurgie au plasma selon l'une quelconque des revendications précédentes, **caractérisé en ce que** la coiffe (15) est arrondie à son extrémité distale dans la direction axiale.

6. Instrument de chirurgie au plasma selon l'une quelconque des revendications précédentes, **caractérisé en ce que** l'électrode (9) est fixée au niveau de la coiffe (15) avec maintien de la distance minimum prédéterminée et peut être raccordée via un dispositif de raccordement électrique (7, 8) à l'intérieur ou au niveau de la coiffe (15) à une ligne électrique (4, 5), qui est adaptée pour être poussée à travers le dispositif d'alimentation (1).

7. Instrument de chirurgie au plasma selon la revendication 6, **caractérisé en ce que** le dispositif de raccordement électrique (7, 8) a un élément de résistance (7) monté directement en amont de l'électrode (9) et une douille de raccordement (8) montée directement en amont de l'élément de résistance (7) pour la ligne électrique (4, 5).

8. Instrument de chirurgie au plasma selon la revendication 7, **caractérisé en ce que**
l'élément de résistance (7) et de préférence la douille de raccordement (8) sont montés par le biais d'une fixation commune sur la coiffe (15) et de préférence sur le capot d'application (10) ou sur l'embout (6).

9. Instrument de chirurgie au plasma selon l'une quelconque des revendications précédentes, **caractérisé en ce que** des marquages/graduations/une échelle (13) sont/est agencé(e)s dans la zone de l'ouverture de traitement (12) vers le tissu cible, pour l'évaluation de la taille pendant un traitement pour des objets, qui sont visibles au niveau du tissu cible à l'intérieur de l'ouverture (12).

10. Instrument de chirurgie au plasma selon l'une quelconque des revendications précédentes, **caractérisé en ce que**
la coiffe (15) contient un dispositif de mesure de température, qui est réalisé pour mesurer une température intérieure du volume d'espace et émettre des informations pouvant être traitées.
